(19) Europäisches Patentamt
European Patent Office
Office européen des brevets

(11) Veröffentlichungsnummer: **0 100 018**
**B1**

# (12) EUROPÄISCHE PATENTSCHRIFT

(45) Veröffentlichungstag der Patentschrift: **02.04.86**

(51) Int. Cl.[4]: **C 07 D 277/42,** C 07 D 417/04, C 07 D 417/06

(21) Anmeldenummer: **83106775.6**

(22) Anmeldetag: **11.07.83**

## (54) Verfahren zur Herstellung von 2-N,N-disubstituierten Aminothiazolen.

(30) Priorität: **22.07.82 DE 3227329**

(43) Veröffentlichungstag der Anmeldung: **08.02.84 Patentblatt 84/6**

(45) Bekanntmachung des Hinweises auf die Patenterteilung: **02.04.86 Patentblatt 86/14**

(84) Benannte Vertragsstaaten: **CH DE FR GB IT LI**

(56) Entgegenhaltungen:
**EP - B - 0 017 769**
**DE - A - 1 812 982**
**DE - A - 2 816 505**
**DE - A - 2 922 523**
**US - A - 4 307 106**

(73) Patentinhaber: **BASF Aktiengesellschaft, Carl-Bosch-Strasse 38, D-6700 Ludwigshafen (DE)**

(72) Erfinder: **Seybold, Guenther, Dr., Friedrich-Ebert-Strasse 14, D-6708 Neuhofen (DE)**

**EP 0 100 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Verbindungen der allgemeinen Formel I

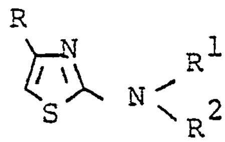 (I)

in der

R ggf. substituiertes Alkyl, Alkenyl, Aryl oder Hetaryl, und

$R^1$ und $R^2$ unabhängig voneinander ggf. substituiertes Alkyl oder Aryl oder zusammen mit dem Stickstoff ein heterocyclischer Ring sind, das dadurch gekennzeichnet ist, dass man Verbindungen der allgemeinen Formel II

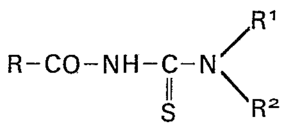 (II)

im wässerig-alkalischen Medium mit wasserlöslichen Monohalogenessigsäuresalzen umsetzt.

Alkylreste R haben z. B. 1 bis 10 C-Atome und können beispielsweise durch Fluor, Chlor, Brom, Hydroxy, $C_1$- bis $C_4$-Alkoxy, $C_1$- bis $C_4$-Alkylmercapto, Phenoxy, Phenylmercapto, Nitro, $C_2$- bis $C_4$-Alkanoylamino, $C_1$- bis $C_4$-Dialkylamino, Aryl oder Hetaryl substituiert sein.

Alkenylreste haben vorzugsweise bis zu 4 C-Atome.

Einzelne Reste sind z. B. $CH_3$, $C_2H_5$, $C_3H_7$, $C_4H_9$, $C_5H_{11}$, $C_6H_{13}$, $C_7H_{15}$, $CH_2CH{=}CH_2$, $CH_2{-}CH{=}CH{-}CH_3$, $CH_2C_6H_5$, $CH_2Cl$, $C_2H_4Cl$, $CH_2OH$, $CH_2N(CH_3)_2$, $C_2H_4N(CH_3)_2$, $CH_2OCH_3$, $CH_2OC_2H_5$, $CH_2SCH_3$, $CH_2SC_2H_5$, $CH_2SC_6H_5$, $CH_2NHCOCH_3$,

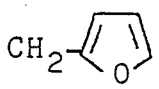 , 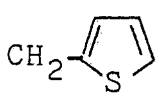 , 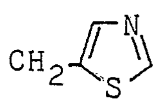 ,

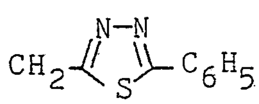 oder 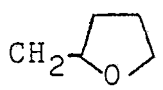 .

Arylreste R sind beispielsweise durch $C_1$- bis $C_8$-Alkyl, $C_1$- bis $C_4$-Alkoxy, Dialkylamino, β-Cyanethoxy, β-$C_1$- bis $C_8$-Alkoxycarbonylethoxy, $C_1$- bis $C_4$-Alkyl- oder Phenylmercapto, Chlor, Brom, Nitro oder $C_1$- bis $C_4$-Alkanoylamino, wie Acetylamino, Propionylamino oder Butyrylamino ein- oder mehrfach substituiertes Phenyl oder Naphthyl.

Einzelne Reste R sind beispielsweise: $C_6H_5$, $C_6H_4Cl$, $C_6H_4Br$, $C_6H_4CH_3$, $C_6H_3ClCH_3$, $C_6H_4NO_2$, $C_6H_3ClNO_2$, $C_6H_4OCH_3$, $C_6H_4OC_2H_5$, $C_6H_4OC_6H_5$, $C_6H_3(OCH_3)_2$, $C_6H_3(CH_3)_2$, $C_6H_2(OCH_3)_3$, $C_6H_4N(CH_3)_2$, $C_6H_4N(C_2H_5)_2$, $C_6H_4C_4H_9$, $C_{10}H_7$,

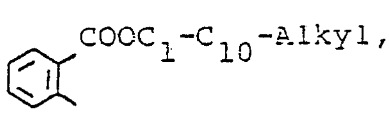

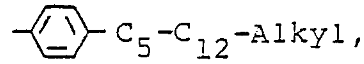 , 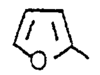 ,

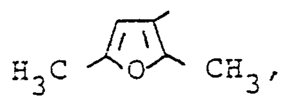 , 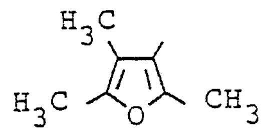 ,

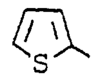 , 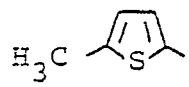 , 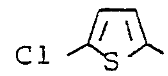

oder 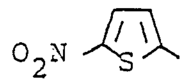 .

Reste $R^1$ und $R^2$ sind z. B. $C_1$- bis $C_8$-Alkylreste, die noch durch Sauerstoff unterbrochen und durch Hydroxy, Cyan, Chlor, $C_1$- bis $C_4$-Alkoxy, $C_1$- bis $C_4$-Alkanoyloxy, $C_1$- bis $C_4$-Alkoxycarbonyl, $C_1$- bis $C_4$-Alkanoylamino, Acetyl, $C_1$- bis $C_4$-Alkylaminocarbonyloxy, Arylaminocarbonyloxy, $C_1$- bis $C_4$-Alkoxycarbonyloxy oder Phenoxycarbonyloxy substituiert sein können, Allyl, Methallyl, Propargyl, Cyclohexyl, Phenyl-$C_1$- bis $C_5$-alkyl, Phenoxyethoxypropyl, Phenyl, Methoxyphenyl, Ethoxyphenyl oder Tolyl.

Einzelne Reste sind neben den bereits genannten beispielsweise:

Methyl, Ethyl, Propyl, Butyl, β-Hydroxyethyl, β-Methoxyethyl, γ-Methoxypropyl, β-Cyanethyl, β-Carbomethoxyethyl, β-Carboethoxyethyl, β-Acetoxyethyl, β-Ethoxycarbonylethyl, γ-Acetylaminopropyl, Phenoxycarbonyloxyethyl, Butylaminocarbonyloxyethyl, Benzyl, β-Phenylethyl.

$R^1$ und $R^2$ zusammen mit dem Stickstoff sind z. B. Pyrrolidino, Piperidino, Morpholino, Piperazino, γ-Methylpiperazino, Hexamethylenimino oder Thiomorpholino-S-dioxid.

Das erfindungsgemässe Verfahren wird zweckmässigerweise so durchgeführt, dass man die Verbindungen der Formel II mit äquimolaren oder grösseren Mengen (bis ~1,5 mol) der Halogenessigsäuresalze in wässerig-alkalischer Lösung 3 bis 8, vorzugsweise 4 bis 6 h, auf 50 bis 110, vorzugsweise 80 bis 95° C, erhitzt.

Die Verbindungen der Formel I scheiden sich in der Regel als Öl ab und können wie üblich, z. B. Extraktion, Phasentrennung oder Destillation, gewonnen werden.

Als Halogenessigsäuresalze eignen sich z. B. die wasserlöslichen Erdalkali- oder Ammoniumsalze und insbesondere die Alkalisalze, von denen das Na-Salz bevorzugt ist. Als wässerig-alkalisches Medium ist z. B. verdünnte Natronlauge (ungefähr 3-15%ig) geeignet. Anstelle der Monohalogenessigsäuresalze kann man in vielen Fällen auch die entsprechenden Säuren verwenden und das Salz *in situ* durch Zugabe der Base herstellen.

Eine besonders bevorzugte Ausführungsform des erfindungsgemässen Verfahrens besteht darin, dass man die Verbindungen der Formel II ohne Isolierung aus Säurehalogeniden der Formel

$$R{-}CO{-}Hal \qquad (Hal{=}Cl, Br)$$

durch Umsetzung mit Alkali- oder Ammonium-

Rhodaniden und anschliessende Zugabe von Aminen der Formel

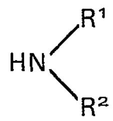

in einem Lösungsmittel wie Aceton, Acetonitril oder Cyclohexanon herstellt, anschliessend Wasser, Alkali und Monohalogenessigsäure zusetzt und das Lösungsmittel entfernt.

Einzelheiten der Herstellung können den Beispielen entnommen werden, in denen sich Angaben über Teile und Prozente, sofern nicht anders vermerkt, auf das Gewicht beziehen.

Die Verbindungen der Formel I sind wertvolle Zwischenprodukte zur Herstellung von Farbstoffen, beispielsweise sind sie als Kupplungskomponenten geeignet, siehe z. B. DE-OS Nr. 2816505 oder EP Nr. 17769.

Aus der DDR-Patentschrift Nr. 112760 ist schon ein Verfahren zur Herstellung von Thiazolen bekannt, nach dem jedoch keine in 5-Stellung unsubstituierten Thiazole gewonnen werden können.

*Beispiel 1:*

In die gut gerührte Mischung von 15 Teilen Aceton und 9,75 Teilen Natriumrhodanid werden bei 20-30° C unter Wasserkühlung innerhalb von 60-90 min 16,86 Teile Benzoylchlorid zugetropft. Nach einstündigem Rühren bei Raumtemperatur werden unter Wasserkühlung 9,63 Teile Diethylamin so zugetropft, dass die Temperatur 40° C nicht übersteigt (Zutropfzeit: 60-90 min). Anschliessend wird bei 40° C 2 h nachgerührt und der Ansatz mit 45 Teilen Wasser verdünnt. In der Reaktionslösung werden 13,62 Teile Chloressigsäure und 21 Teile 50%ige Natronlauge gegeben. Die Temperatur steigt dabei auf 50-60° C. Nun wird langsam unter Abdestillieren eines Aceton-Wassergemisches (Menge des Destillats: 10-12 Teile) auf 90-95° C erhitzt und bei dieser Temperatur 12 h gerührt. Man kühlt auf 70-75° C und lässt die wässerige (untere) Phase ab. Die obere, ölige Phase wird abgefüllt.

Ausbeute: 24,5 Teile 2-Diethylamino-4-Phenylthiazol ≙80% der Theorie.

*Beispiel 2:*

In die gut gerührte Mischung von 700 Teilen Aceton und 374 Teilen Natriumrhodanid werden bei 20-30° C unter Wasserkühlung innerhalb von 60-90 min 774 Teile rohes p-Methoxybenzoylchlorid ber. 100% zugetropft. Nach einstündigem Nachrühren bei Raumtemperatur werden unter Wasserkühlung 370 Teile Diethylamin so zugetropft, dass die Temperatur 40° C nicht übersteigt (Zeitaufwand 60-90 min). Anschliessend wird bei 40° C 2 h nachgerührt und der Ansatz mit 1725 Teilen Wasser verdünnt. Man gibt 522 Teile Chloressigsäure und 800 Teile Natronlauge 50%ig zu. Die Temperatur steigt auf 50-60° C. Nun wird langsam unter Abdestillieren eines Aceton-Wassergemisches auf 90-95° C Innentemperatur erhitzt und bei dieser Temperatur 12 h gerührt. Man kühlt auf 70-80° C und lässt bei dieser Temperatur die untere (wässerige) Phase ab. Das ölige Wertprodukt wird abgefüllt.

Ausbeute: 1093 Teile 2-Diethylamino-4-p-methoxyphenylthiazol

Gehalt laut GC 96% ≙88% der Theorie.

*Beispiel 3:*

In die gut gerührte Mischung von 500 Teilen Aceton und 324 Teilen Natriumrhodanid werden unter Wasserkühlung bei 20-30° C 562 Teile Benzoylchlorid im Verlauf von 1 bis 1,5 h eingetropft. Nach halbstündigem Nachrühren bei Raumtemperatur werden unter Wasserkühlung bei 20-30° C 404 Teile N-Butyl-N-ethylamin zugetropft. Bei Raumtemperatur wird 2 h kräftig nachgerührt und dann 1000 Teile Wasser zugegeben. Nach kurzem Aufrühren wird die wässerige (untere) Phase abgelassen, 1000 Teile Wasser und 454 Teile Chloressigsäure hinzugefügt. Nun lässt man 640 Teile Natronlauge (50%ig) zulaufen; die Temperatur erhöht sich dabei auf 50-60° C. Es wird nun auf Rückfluss erhitzt und so viel Aceton-Wassergemisch (ca. 200 Teile) abdestilliert, bis die Innentemperatur auf 90° C steigt. Auf dieser Temperatur wird 6-8 h gehalten. Nach dem Abkühlen wird die wässerige (untere) Phase abgelassen und das Produkt abgefüllt.

Ausbeute: 1109 Teile gelbliches Öl, enthält laut GC-Analyse

840 Teile 2-N-Ethyl-N-butylamino-4-phenylthiazol ≙81% der Theorie.

*Beispiel 4:*

In eine Mischung von 66,4 Teilen Natriumrhodanid und 126 Teilen Aceton werden unter starkem Rühren bei −5 bis 0° C innerhalb von 1 h 116,8 Teile Thiophen-2-carbonsäurechlorid eingetropft. Man rührt anschliessend 30 min bei 0° C nach und tropft innerhalb von 1-1,5 h bei −2-+2° C 60 Teile Diethylamin zu. Nach einer Nachrührzeit von 1 h bei 0° C und 30 min bei 5-10° C gibt man 320 Teile Wasser, 83 Teile Chloressigsäure und 135 Teile NaOH 50% zu. Man erhitzt langsam auf 90° C und destilliert dabei Aceton ab. Nachrührzeit bei 90-95° C: 4 h. Anschliessend lässt man abkühlen und trennt bei 70° C das Produkt als obere ölige Phase ab.

Ausbeute: 170 Teile Rohprodukt
=153 Teile 4-Thienyl-2-diethylaminothiazol
≙80% der Theorie.

Analog Beispiel 1 wurden auch die folgenden Verbindungen hergestellt.

| Beispiel | Säurechlorid | Amin | Thiazol |
|---|---|---|---|
| 5 | 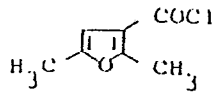 | HNEt₂ | 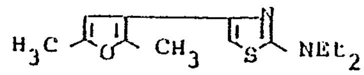 |
| 6 | 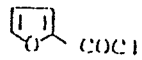 | HNEt₂ | 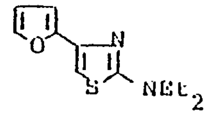 |
| 7 | 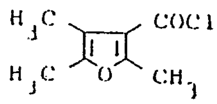 | HNEt₂ | 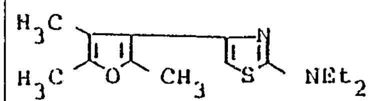 |
| 8 | 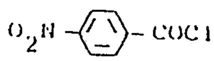 | $HN(C_4H_9)_2$ | 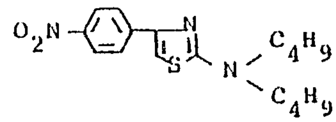 Schmp. 76° C |
| 9 | 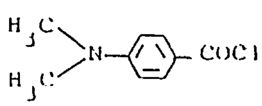 | $HN(C_2H_5)_2$ | 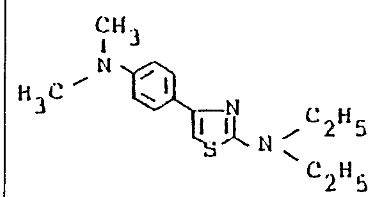 Schmp. 90-92° C |
| 10 | 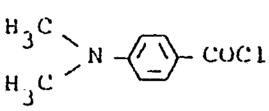 | 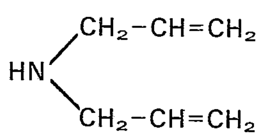 | 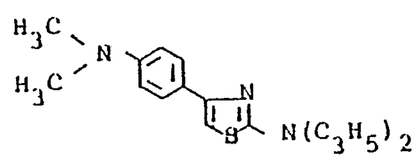 |
| 11 | 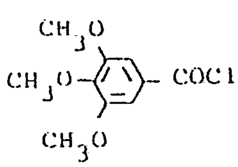 | $HN(C_2H_5)_2$ | 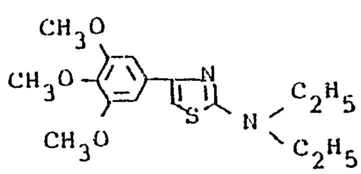 Schmp. 112° C |
| 12 | $(CH_3)_3C-COCl$ | $HN(C_2H_5)_2$ | 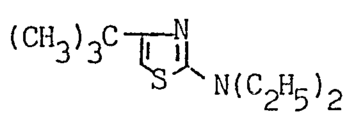 |
| 13 | $(CH_3)_3C-COCl$ | $HN(CH_3)_2$ | 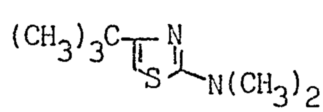 |
| 14 | 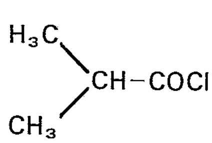 | $HN(C_2H_5)_2$ | 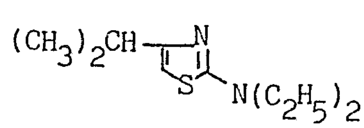 |

| Beispiel | Säurechlorid | Amin | Thiazol |
|---|---|---|---|
| 15 | $C_6H_5CH_2-COCl$ | 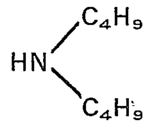 | 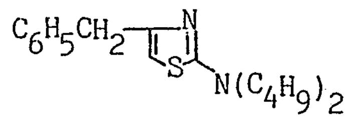 |
| 16 | $C_2H_5O-CH_2-COCl$ | $HN(C_2H_5)_2$ | 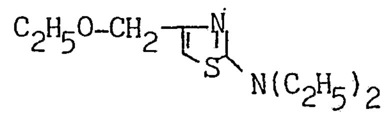 |
| 17 | 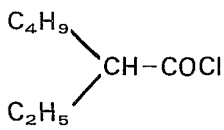 | $HN(C_2H_5)_2$ | 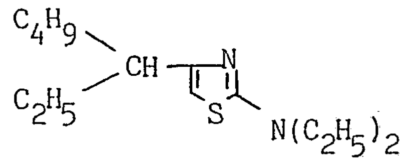 |
| 18 | $CH_3-S-CH_2-CH_2COCl$ | 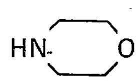 | 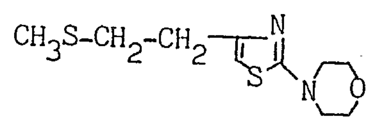 |
| 19 | $C_6H_5-CH=CH-COCl$ | $HN(C_2H_5)_2$ | 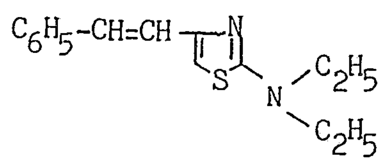 |
| 20 | 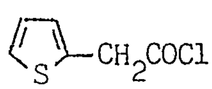 | $HN(C_2H_5)_2$ | 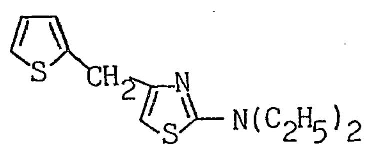 |
| 21 | 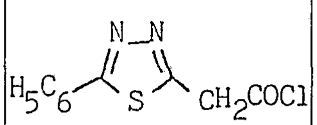 | $HN(C_2H_5)_2$ | 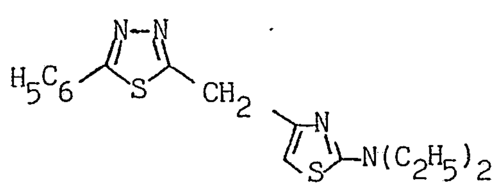 |

## Patentansprüche

1. Verfahren zur Herstellung von 2-N,N-disubstituierten Aminothiazolen der allgemeinen Formel (I)

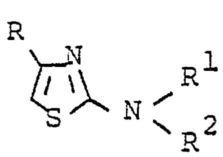

(I)

in der

R ggf. substituiertes Alkyl, Alkenyl, Aryl oder Hetaryl, und

$R^1$ und $R^2$ unabhängig voneinander ggf. substituiertes Alkyl oder Aryl oder zusammen mit dem Stickstoff ein heterocyclischer Ring sind, dadurch gekennzeichnet, dass man Verbindungen der allgemeinen Formel (II)

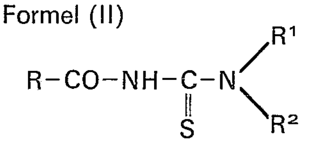

(II)

im wässerig-alkalischen Medium mit wasserlöslichen Monohalogenessigäuresalzen umsetzt.

2. Verfahren gemäss Anspruch 1, dadurch gekennzeichnet, dass man Verbindungen herstellt, bei denen

R ggf. durch Chlor, $C_1$- bis $C_4$-Alkyl, $C_1$- bis $C_4$-Alkoxy oder $C_1$- bis $C_4$-Dialkylamino substituiertes Phenyl, ggf. durch Chlor oder Methyl substituiertes Thienyl, $C_1$- bis $C_8$-Alkyl oder Benzyl ist.

3. Verfahren gemäss Anspruch 1, dadurch gekennzeichnet, dass man Verbindungen herstellt, bei denen

$R^1$ und $R^2$ ggf. durch $C_1$- bis $C_4$-Alkoxy oder $C_1$- bis $C_4$-Alkoxycarbonyl substituiertes $C_1$- bis $C_8$-Alkyl, Benzyl, Cyclohexyl oder Phenyl oder zusammen mit dem Stickstoff Piperidino, Pyrrolidino oder Morpholino sind.

4. Verfahren gemäss Anspruch 1, dadurch gekennzeichnet, dass man die Verbindung der Formel (II) *in situ* durch Umsetzung von Säurehalogeniden der Formel

R−COCl

mit Alkali- oder Ammoniumrhodaniden und anschliessendem Zusatz von Aminen der Formel

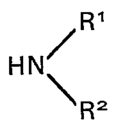

herstellt.

## Revendications

1. Procédé pour la préparation de 2-aminothiazoles N,N-disubstitués de formule générale

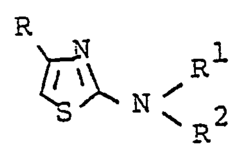
(I)

dans laquelle

R est un radical alkyle, alcényle, aryle ou hétéroaryle éventuellement substitué,

$R^1$ et $R^2$, considérés indépendamment l'un de l'autre, représentent chacun un radical alkyle ou aryle éventuellement substitué ou forment avec l'atome d'azote un noyau hétérocyclique, caractérisé en ce qu'on fait réagir des composés de formule générale

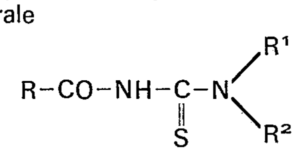
(II)

dans un milieu hydroalcalin, avec des sels d'acides monohalogénacétiques solubles dans l'eau.

2. Procédé selon la revendication 1, caractérisé en ce qu'on prépare des composés dans lesquels

R est un radical phényle éventuellement substitué par un atome de chlore, par un radical alkyle en $C_1$ à $C_4$, alcoxy en $C_1$ à $C_4$ ou dialkylamino en $C_1$ à $C_4$, un radical thiényle éventuellement substitué par un atome de chlore ou par un radical méthyle, un radical alkyle en $C_1$ à $C_8$ ou un radical benzyle.

3. Procédé selon la revendication 1, caractérisé en ce qu'on prépare des composés dans lesquels

$R^1$ et $R^2$ sont des radicaux alkyle en $C_1$ à $C_8$ éventuellement substitués par des groupes alcoxy en $C_1$ à $C_4$ ou par des groupes alcoxycarbonyle en $C_1$ à $C_4$, benzyle, cyclohexyle ou phényle ou forment, avec l'atome d'azote, des groupements pipéridine, pyrrolidino ou morpholino.

4. Procédé selon la revendication 1, caractérisé en ce qu'on prépare le composé de formule (II) *in situ* par réaction d'halogénures d'acide de formule

R−COCl

avec des rhodanures de métal alcalin ou d'ammonium, puis par addition d'amines de formule

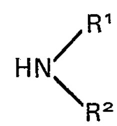

## Claims

1. A process for the preparation of an N,N-disubstituted 2-aminothiazole of the general Formula (I)

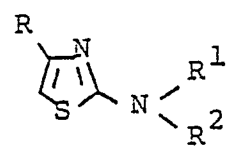
(I)

where

R is unsubstituted or substituted alkyl, alkenyl, aryl or hetaryl, and

$R^1$ and $R^2$ independently of one another are unsubstituted or substituted alkyl or aryl, or, together with the nitrogen, form a heterocyclic ring, wherein a compound of the general Formula (II)

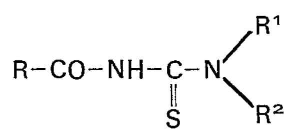
(II)

is reacted with a water-soluble monohaloacetate in an aqueous alkaline medium.

2. A process as claimed in Claim 1, wherein a compound is prepared in which

R is phenyl which is unsubstituted or substituted by chlorine, $C_1$-$C_4$-alkyl, $C_1$-$C_4$-alkoxy or $C_1$-$C_4$-dialkylamino, or is $C_1$-$C_8$-alkyl, benzyl or thienyl which is unsubsituted or substituted by chlorine or methyl.

3. A process as claimed in Claim 1, wherein a compound is prepared in which

$R^1$ and $R^2$ are each benzyl, cyclohexyl, phenyl or $C_1$-$C_8$-alkyl which is unsubstituted or substituted by $C_1$-$C_4$-alkoxy or $C_1$-$C_4$-alkoxycarbonyl, or $R^1$ and $R^2$ together with the nitrogen form piperidino, pyrrolidino or morpholino.

4. A process as claimed in Claim 1, wherein the compound of the Formula (II) is prepared *in situ* by reacting an acid halide of the formula

R−COCl

with an alkali metal thiocyanate or ammonium thiocyanate and then adding an amine of the formula